# EUROPEAN PATENT SPECIFICATION

(11) **EP 4 115 928 B1**
(45) Date of publication and mention of the grant of the patent: **18.02.2026**
(21) Application number: 21768244.2
(22) Date of filing: 08.03.2021
(51) Int. Cl.: A61M 5/24, A61M 5/28, A61M 5/315, A61M 5/32, A61J 1/20, A61M 5/31, A61M 5/34

(54) **SYRINGE SET, SYRINGE, AND SET**
SPRITZENSET, SPRITZE UND SET
ENSEMBLE SERINGUE, SERINGUE ET ENSEMBLE

(30) Priority: 09.03.2020 JP 2020039723
(43) Date of publication of application: 11.01.2023
(73) Proprietor: Terumo Kabushiki Kaisha, Tokyo 151-0072 (JP)
(72) Inventor: OKIHARA, Hitoshi, Fujinomiya-shi, Shizuoka 418-0004 (JP)
(74) Representative: Casalonga
(86) International application number: PCT/JP2021/008877
(87) International publication number: WO 2021/182370

(56) References cited:
- WO-A1-2016/181826
- JP-A- 2011 194 045
- JP-A- 2014 079 331
- JP-A- 2014 200 504
- JP-A- 2014 200 505
- JP-A- 2014 200 505
- US-A1- 2012 123 382
- US-A1- 2016 095 988
- US-A1- 2018 117 264

## Description

### Technical Field

The present invention relates to a syringe set.

### Background Art

An injection to be used after two drugs that are separately stored are mixed is used. Such an injection is provided, for example, in such a manner that one drug is enclosed in a vial container or a syringe as a powder and the other drug is enclosed in a syringe for injection as a solution such as water for injection or physiological saline (JP 2013-132349 A).
JP 2014 200 505 A provides a medicine preparation device which includes: an outer cylinder for a syringe having a nozzle part which can be connected to a female connecting part having a female luer taper conforming to ISO standards at the tip; an adapter which is detachably attached to the tip of the outer cylinder and impedes other medical devices from being connected to the nozzle part; and a medicine container having a cylindrical tip part. The outer cylinder to which the adapter is attached can be connected to the cylindrical tip part of the medicine container using a medicine container connecting part of the adapter, and after connection, the medicine container along with the adapter can be detached from the outer cylinder, and the nozzle part which can be connected to the female connecting part is exposed by the detachment of the adapter.
US 2018/ 117264 A1 shows a drug delivering apparatus and a pre-filled syringe. Thereby a drug delivering apparatus includes a drug container, a pre-filled syringe including a cylindrical container having a nozzle portion and an injection needle attachment portion, and a connecting tool configured to allow communication between the inside of the cylindrical container and the inside of the drug container. The connecting tool further includes a nozzle insertion portion. The pre-filled syringe further includes a cap member configured to be detachably fitted on the injection needle attachment portion, the cap member having an opening into which the nozzle insertion portion can be inserted. The injection needle attachment portion has a guiding groove in the inner periphery thereof. In the state where the cap member is fitted on the injection attachment portion, at least a part of the edge of the opening is located on the inner side relative to the bottom portion of the guiding groove.
JP 2014 200 504 A provides a medicine preparation device which includes: an outer cylinder having a tip opening part which cannot be connected to a female connecting part conforming to the ISO standards for other medical devices; a medicine container having a cylindrical tip part; and an adapter detachably attached to the cylindrical tip part of the medicine container. The adapter includes: an outer cylinder connecting part which can be connected to the tip opening part of the outer cylinder; and a nozzle part which can communicate with the inside of the medicine container and which can be connected to the female connecting part conforming to the ISO standards. The medicine container can be connected to the tip opening part of the outer cylinder using the outer cylinder connecting part of the adapter, and after the connection, the nozzle part conforming to the ISO standards for the adapter connected to the outer cylinder is exposed by detaching the medicine container from the adapter.
US 2012/ 123382 A1 shows a device capable of preventing an operation of establishing communication between a syringe and a vial from being forgotten by error. Holding means constituting a connection device includes: a syringe holding member to be attached to a syringe; a cannula holding member which includes a double-head cannula, is located at a retracted position retracted with respect to the syringe holding member in the pre-use state, and is advanced to an advanced position abutting on the syringe holding member in the use state; and separation preventing means which engages the syringe to prevent separation of the syringe from the syringe holding member in the state where the cannula holding member is located at the retracted position. When the cannula holding member is located at the advanced position, the separation preventing means releases an engagement state with the syringe to permit the separation of the syringe from the syringe holding member.
US 2016/ 095988 A1 shows a plug for placing on a connection element of a medical syringe, which is connectable to the connection element, the plug comprising a through-duct and being formed free of further connectivity or connection elements.

The injection is prepared by connecting one syringe (or vial) to the other syringe for injection and mixing the two drugs while operating a plunger of the syringe for injection. Then, the prepared injection is transferred to the syringe for injection, the syringe (or vial) is removed, and an injection needle is attached to the syringe for injection to perform administration.

### Summary of Invention

The invention is defined by the appended claims.

By the way, work of preparing an injection is performed not only by a medical institution but also by a patient at home in some cases. When the patient himself/herself administers an injection, there is a case where the patient forgets mixing drugs. In such a case, there is a possibility that the patient erroneously recognizes a drug (for example, a solution such as an injection solution or physiological saline) enclosed in a syringe for injection as a prepared drug, attaches an injection needle to a syringe and erroneously administers the drug without performing mixing operation.

It is therefore an object of one embodiment to provide a syringe set capable of preventing erroneous administration of an injection that is to be prepared by mixing drugs before use, due to forgetting of mixing the drugs.

According to one aspect of the following disclosure, there is provided a syringe set including: a first syringe including a barrel that stores a liquid first drug and a first connection port that is provided at a distal end of the barrel and communicates with inside of the barrel; and a drug container that stores a second drug and includes a second connection port that configured to be attachable to and detachable from the first connection port, the first connection port including an injection needle attachment structure to which an injection needle is attachable, and a blocking member that is disposed in the injection needle attachment structure and configured to block attachment of the injection needle to the injection needle attachment structure, when the second connection port is connected to the first connection port, the blocking member being connected to the second connection port side, and when the second connection port to which the blocking member is connected is detached from the first connection port, the blocking member being detached from the first connection port so as to allow the injection needle to be attached to the injection needle attachment structure. The injection needle attachment structure comprises: a tubular lock portion in which a needle connection screw into which the injection needle is to be screwed is formed on an inner peripheral portion; and a luer tip formed inside the lock portion and having a through hole communicating with the inside of the barrel, and the blocking member is disposed between the lock portion and the luer tip. A male screw is formed on an outer peripheral portion of the lock portion of the first connection port. The second connection port comprises: an outer tubular portion having a female screw on an inner periphery, the female screw being capable of being screwed into the male screw on the outer periphery portion of the lock portion. An inner tubular portion, that is formed inside the outer tubular portion, has a communication hole communicating with inside of the drug container, is inserted between the lock portion and the luer tip, and stores the luer tip in the communication hole.

According to the syringe set from the above viewpoint, the injection needle is not attached if mixing is forgotten, so that it is possible to prevent erroneous administration due to forgetting of mixing.

### Brief Description of Drawings

Fig. 1 is a plan view of a syringe set according to a first embodiment.
Fig. 2A is a cross-sectional view of a first syringe of Fig. 1, and Fig. 2B is an enlarged cross-sectional view of a distal end portion of the first syringe of Fig. 2A.
Fig. 3A is a cross-sectional view of a second syringe of Fig. 1, and Fig. 3B is an enlarged cross-sectional view of a distal end portion of the second syringe of Fig. 3A.
Fig. 4A is a cross-sectional view of a state in which a cap of the first syringe is removed, and Fig. 4B is a cross-sectional view of a state in which a cap of the second syringe is removed.
Fig. 5 is a cross-sectional view illustrating action of a blocking member of a first connection port.
Fig. 6 is a cross-sectional view illustrating a state in which a second connection port is connected to the first connection port and drugs are mixed between the first syringe and the second syringe.
Fig. 7 is a cross-sectional view of a state in which the second connection port is removed from the first connection port after the drugs are mixed.
Fig. 8 is a cross-sectional view of a state in which an injection needle is attached to the first connection port.
Fig. 9 is a side view of a blocking member according to a modification of the first embodiment.
Fig. 10 is an exploded perspective cross-sectional view of a first syringe and a second syringe of a syringe set according to a second embodiment.
Fig. 11 is a cross-sectional view of a state in which a second connection port is connected to a first connection port of Fig. 10.
Fig. 12 is a perspective cross-sectional view of a first syringe and a second syringe of a syringe set according to a third embodiment.
Fig. 13 is a cross-sectional view of a state in which a first connection port is connected to a second connection port in a syringe set according to a fourth embodiment.
Fig. 14 is a perspective view of a first syringe and a vial adapter of a syringe set according to a fifth embodiment.
Fig. 15 is a cross-sectional view of a state in which the vial adapter is connected to the first syringe of Fig. 14.

### Description of Embodiments

Hereinafter, the present invention will be described in detail using preferred embodiments of the present invention with reference to the accompanying drawings.

### (First Embodiment)

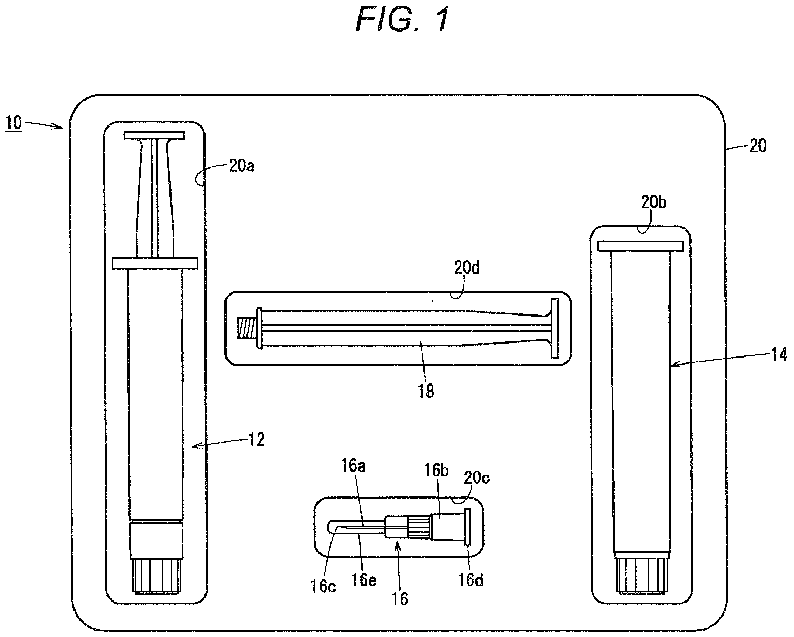

A syringe set 10 according to the present embodiment is used for an injection to be prepared by mixing two types of drugs that are separately stored and includes a first syringe 12 (syringe), a second syringe 14, and an injection needle 16 as illustrated in Fig. 1. The first syringe 12 and the second syringe 14 store different types of drugs, respectively.

The first syringe 12, the second syringe 14, and the injection needle 16 are provided to a user in a state where they are accommodated in a tray 20 having a plurality of accommodation recesses 20a to 20d. Although not particularly limited, a second plunger 18 of the second syringe 14 is separated from the second syringe 14 and accommodated in the accommodation recess 20d. The second plunger 18 may be assembled to the second syringe 14 in advance.

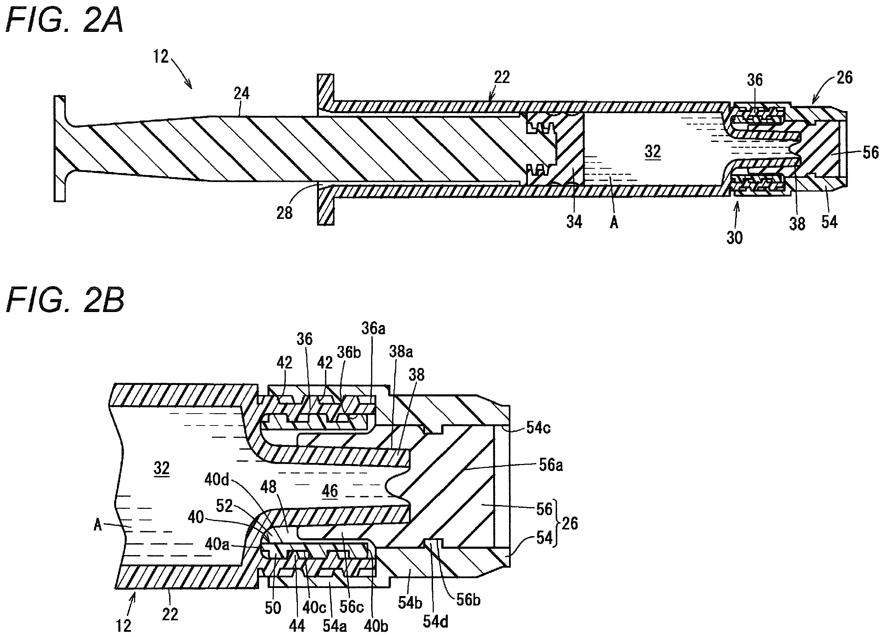

As illustrated in Fig. 2A, the first syringe 12 includes a first barrel 22, a first plunger 24, and a first cap 26. Among them, the first barrel 22 is formed in a cylindrical shape, a first opening portion 28 is formed on a proximal end side, and a first connection port 30 is formed on a distal end side. Inside of the first barrel 22 is a first storage chamber 32 that stores a drug. The first barrel 22 is formed with a transparent resin so as to allow a state of the first drug A inside the first barrel 22 to be visually recognized.

A first gasket 34 is inserted into the first barrel 22, and a proximal end side of the first storage chamber 32 is sealed by the first gasket 34. The first drug A is sealed in the first storage chamber 32. Although not particularly limited, the first drug A can be, for example, a liquid such as water (injection water) or physiological saline for diluting the other second drug B.

The first plunger 24 is inserted from the first opening portion 28 on the proximal end side of the first barrel 22. The first plunger 24 is connected to the first gasket 34. The first plunger 24 may be configured to be attached to the first gasket 34 immediately before use.

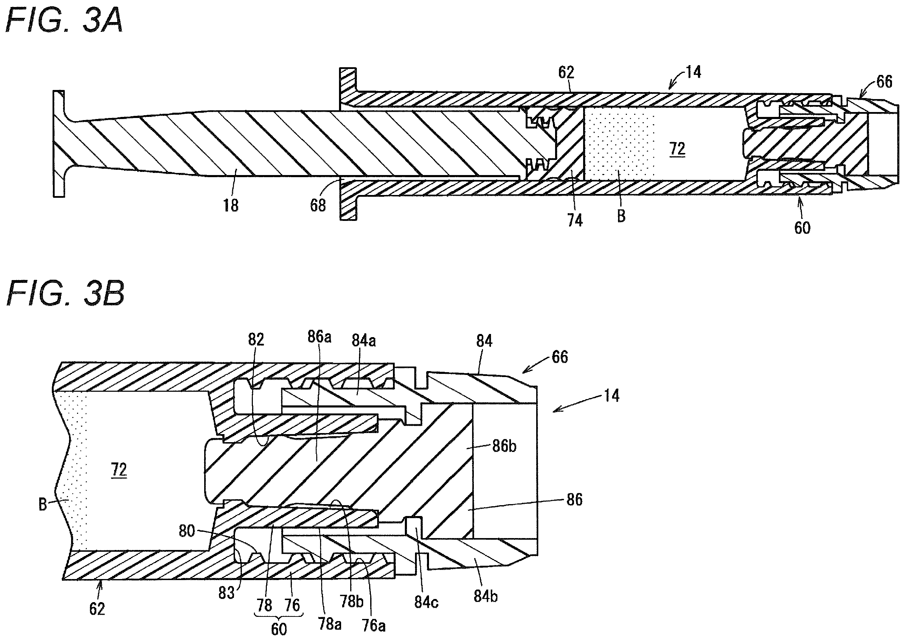

The first connection port 30 is a structure for connection to a second connection port 60 (see Fig. 3B) of the second syringe 14 which will be described later and connection of the injection needle 16. The first connection port 30 includes a lock portion 36 extending in a tubular shape from the vicinity of an outer periphery of the first barrel 22 toward the distal end side of the first barrel 22, and a luer tip 38 extending in a tubular shape from the vicinity of a central axis of the first barrel 22 toward the distal end side of the first barrel 22 inside the lock portion 36. The injection needle attachment structure of the first connection port 30 includes the lock portion 36 and the luer tip 38. In the first connection port 30 in an unused state, a blocking member 40 that blocks attachment of the injection needle 16 is disposed between the lock portion 36 and the luer tip 38.

A male screw 42 is formed on an outer peripheral portion 36a of the lock portion 36. The male screw 42 is constituted with one or more spiral screw structures. Further, a needle connection screw 44 for screwing and attaching the injection needle 16 is formed on an inner peripheral portion 36b of the lock portion 36. The needle connection screw 44 is constituted as a spiral screw structure having the same pitch as the male screw 42.

The luer tip 38 is formed to protrude longer toward the distal end side in an axial direction than the lock portion 36. A through hole 46 penetrating in the axial direction is formed in the vicinity of the central axis of the luer tip 38. A proximal end side of the through hole 46 is opened to the first storage chamber 32 of the first barrel 22, so that the through hole 46 communicates with the first storage chamber 32. An outer peripheral portion 38a of the luer tip 38 is formed in a tapered shape such that an outer diameter gradually decreases toward the distal end side. The outer peripheral portion 38a of the luer tip 38 is formed to have an outer diameter smaller than that of the inner peripheral portion 36b of the lock portion 36, and a gap 48 is formed between the outer peripheral portion 38a and the inner peripheral portion 36b of the lock portion 36. The blocking member 40 that blocks entry of a proximal end of the injection needle 16 is disposed in the gap 48.

The blocking member 40 is formed in a cylindrical shape having an outer diameter and an inner diameter that allow the blocking member 40 to be inserted into the gap 48. A proximal end portion 40a of the blocking member 40 abuts on the distal end of the first barrel 22, and a distal end portion 40b of the blocking member 40 extends to the vicinity of the distal end of the lock portion 36. An outer peripheral portion 40c of the blocking member 40 is provided with a male screw 50 that can be screwed into the needle connection screw 44 of the inner peripheral portion 36b of the lock portion 36. The male screw 50 has a screw structure with the same pitch as the needle connection screw 44 and is screwed into the needle connection screw 44 to prevent the blocking member 40 from falling off to the distal end side.

An inner peripheral portion 40d of the blocking member 40 is formed to have an inner diameter larger than that of the outer peripheral portion 38a of the luer tip 38, and the inner peripheral portion 40d is separated radially outward of the luer tip 38. A columnar gap 52 is formed between the inner peripheral portion 40d of the blocking member 40 and the outer peripheral portion 38a of the luer tip 38. A width (dimension) in a radial direction of the gap 52 is set to a value substantially equal to or slightly smaller than a thickness (dimension in a radial direction) of the inner tubular portion 78 of the second connection port 60 which will be described later.

A first cap 26 is detachably attached to the distal end side of the first connection port 30. The first cap 26 includes a cylindrical main body portion 54 and a sealing member 56 disposed so as to close an inner peripheral side of the main body portion 54. The main body portion 54 includes a twisting portion 54a to be screwed into the male screw 42 of the outer peripheral portion 36a of the lock portion 36. A first cap body portion 54b whose diameter is reduced radially inward is formed on the distal end side of the twisting portion 54a. A seal storage hole 54c having an inner diameter smaller than the inner diameter of the lock portion 36 is formed inside the first cap body portion 54b, and an end portion of the sealing member 56 is stored in the seal storage hole 54c.

The sealing member 56 is formed with an elastically deformable material such as a rubber material, and a distal end portion 56a of the sealing member 56 is stored in the seal storage hole 54c. A recess 56b engaged with a protrusion 54d protruding from the seal storage hole 54c is formed in the distal end portion 56a, and the sealing member 56 is stored in a state where the sealing member 56 is not displaced in the axial direction by the recess 56b being engaged with the protrusion 54d.

A close contact portion 56c formed in a tubular shape is formed on the proximal end side of the sealing member 56. The close contact portion 56c is formed in a bottomed cylindrical shape that can be in close contact with the outer peripheral surface of the luer tip 38. Part of a distal end side of the close contact portion 56c is formed thick and is compressed in the radial direction by the outer peripheral surface of the luer tip 38 and the seal storage hole 54c, so that the close contact portion 56c is reliably brought into close contact with the outer peripheral surface of the luer tip 38. The sealing member 56 seals the through hole 46 at the distal end of the luer tip 38 in an airtight and liquid-tight manner. The distal end side of the first storage chamber 32 of the first barrel 22 is sealed by the sealing member 56.

On the other hand, as illustrated in Fig. 3A, the second syringe 14 includes a second barrel 62, a second plunger 18, and a second cap 66. Among them, the second barrel 62 is formed in a cylindrical shape, a second opening portion 68 is formed on the proximal end side, and a second connection port 60 is formed on the distal end side. Inside of the second barrel 62 is a second storage chamber 72 that stores a drug. A second gasket 74 is inserted into the second barrel 62, and a proximal end side of the second storage chamber 72 is sealed by the second gasket 74. The second barrel 62 is formed with a transparent resin so as to allow a state of the second drug B inside the second barrel 62 to be visually recognized. Although not particularly limited, the second drug B can be, for example, a powder or a liquid.

The second plunger 18 can be inserted from the second opening portion 68 on the proximal end side of the second barrel 62, and the second plunger 18 is connected to the second gasket 74 immediately before use as illustrated in the drawing. The second plunger 18 may be attached to the second gasket 74 in advance.

The second connection port 60 is used for connection to the first connection port 30 (see Fig. 2B) of the first syringe 12. The second connection port 60 includes an outer tubular portion 76 extending in a tubular shape from the vicinity of an outer periphery of the second barrel 62 toward the distal end side of the second barrel 62, and an inner tubular portion 78 extending in a tubular shape from the vicinity of a central axis of the second barrel 62 toward the distal end side of the second barrel 62 inside the outer tubular portion 76.

The outer tubular portion 76 is formed in a cylindrical shape having an inner diameter larger than that of the lock portion 36, and a female screw 80 is formed on an inner peripheral portion 76a of the outer tubular portion 76. The female screw 80 is formed with the same number of stripes and the same pitch as the male screw 42 formed on the outer peripheral portion 36a of the lock portion 36, and the female screw 80 can be screwed into the male screw 42.

The inner tubular portion 78 is formed to be slightly shorter in the axial direction than the outer tubular portion 76. A communication hole 82 penetrating in the axial direction is formed on the inner peripheral side of the inner tubular portion 78. The proximal end side of the communication hole 82 is opened to a second storage chamber 72 of the second barrel 62, so that the communication hole 82 communicates with the second storage chamber 72. An inner peripheral portion 78b of the inner tubular portion 78 is formed in a tapered shape such that an inner diameter gradually decreases toward the proximal end side so as to be in close contact with the outer peripheral portion 38a of the luer tip 38. A thickness (dimension in a radial direction) of the inner tubular portion 78 is set to be equal to or slightly larger than the gap 52 between the inner peripheral portion 40d of the blocking member 40 and the outer peripheral portion 38a of the luer tip 38. A gap 83 is formed between the inner tubular portion 78 and the outer tubular portion 76. The lock portion 36 and the blocking member 40 of the first connection port 30 are inserted into the gap 83.

The second cap 66 is detachably attached to the distal end side of the second connection port 60. The second cap 66 includes a cylindrical main body portion 84 and a seal member 86 disposed so as to close the inner peripheral side of the main body portion 84. The main body portion 84 includes a twisting portion 84a to be screwed into the female screw 80 of the inner peripheral portion 76a of the outer tubular portion 76. A second cap body portion 84b that holds the seal member 86 is formed on the distal end side of the twisting portion 84a. A protrusion 84c protruding inward is formed in the second cap body portion 84b, and the seal member 86 is held by the second cap body portion 84b by the seal member 86 being caught by the protrusion 84c.

The seal member 86 is formed with an elastically deformable material such as a rubber material, and a distal end portion 86b of the seal member 86 is stored in the second cap body portion 84b. A columnar portion 86a is formed on the proximal end side of the seal member 86, and the columnar portion 86a is inserted into the communication hole 82 of the inner tubular portion 78 to seal the communication hole 82 in an airtight and liquid-tight manner. The distal end side of the second storage chamber 72 of the second barrel 62 is sealed by the seal member 86.

The injection needle 16 includes a needle tube 16a and a needle hub 16b that supports the needle tube 16a. The needle tube 16a is formed with a metal such as stainless steel, for example, and a needle tip 16c capable of puncturing the skin is formed at the distal end of the needle tube 16a. The needle hub 16b is formed with, for example, a resin member, and a needle hole communicating with the inside of the needle tube 16a is provided inside the needle hub 16b. The needle hole inside the needle hub 16b is formed in a shape that can be brought into close contact with the luer tip 38 of the first connection port 30. In addition, a flange portion 16d that can be screwed into the needle connection screw 44 of the lock portion 36 is provided at the proximal end of the needle hub 16b. The injection needle 16 is provided in a state where the needle tube 16a is covered with the needle cap 16e.

The syringe set 10 of the present embodiment is configured as described above, and action thereof will be described below.

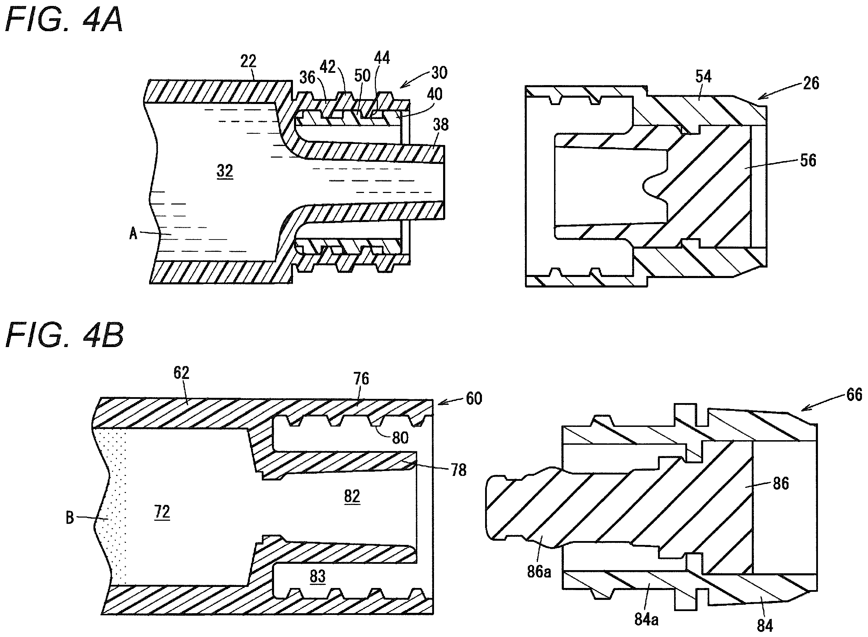

The first syringe 12 and the second syringe 14 of the syringe set 10 are removed from the tray 20 of Fig. 1 by a user. Thereafter, as illustrated in Fig. 4A, the user holds the first syringe 12 and rotates the first cap 26 to detach the first cap 26 from the first connection port 30.

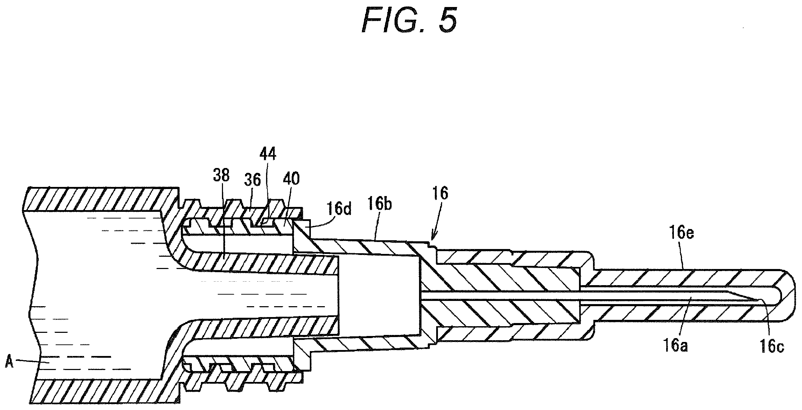

When the first cap 26 is removed from the first connection port 30, the lock portion 36 appears. In this state, the blocking member 40 is disposed on the inner peripheral side of the lock portion 36. As illustrated in Fig. 5, when the user forgets mixing the first drug A and the second drug B and tries to attach the injection needle 16 to the first connection port 30 of the first syringe 12, the proximal end of the needle hub 16b of the injection needle 16 is hindered by the blocking member 40. In order to connect the injection needle 16 to the first connection port 30, it is necessary to screw the flange portion 16d of the injection needle 16 to the needle connection screw 44 of the lock portion 36. In the first syringe 12 of the present embodiment, the injection needle 16 is blocked from proceeding by the blocking member 40, and thus the injection needle 16 is blocked from being screwed into the lock portion 36. This results in preventing the user from attaching the injection needle 16 to the first syringe 12 that stores only the first drug A.

Thereafter, as illustrated in Fig. 4B, the user holds the second syringe 14 and rotates the second cap 66 to detach the second cap 66 from the second connection port 60. As a result, the first syringe 12 can be connected to the second syringe 14.

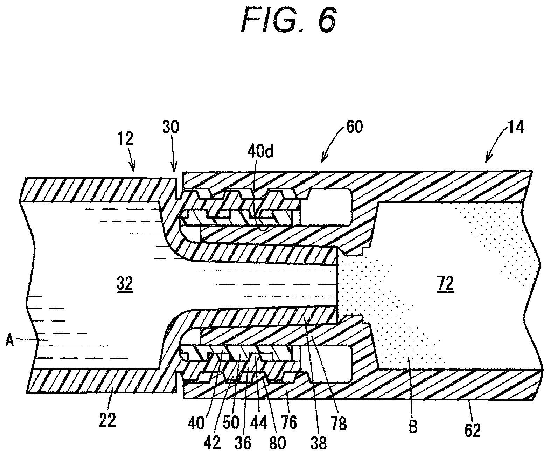

Next, as illustrated in Fig. 6, the user screws the second connection port 60 of the second syringe 14 into the first connection port 30 of the first syringe 12 to connect the first connection port 30 to the second connection port 60. The female screw 80 of the inner peripheral portion 76a of the outer tubular portion 76 of the second connection port 60 and the male screw 42 of the outer peripheral portion 36a of the lock portion 36 of the first connection port 30 are screwed together, whereby the first connection port 30 is connected to the second connection port 60.

In addition, the luer tip 38 of the first connection port 30 is connected in close contact with inside of the communication hole 82 of the inner tubular portion 78 of the second connection port 60 in a liquid-tight and airtight manner. As a result, the first storage chamber 32 of the first syringe 12 communicates with the second storage chamber 72 of the second syringe 14. The inner tubular portion 78 of the second connection port 60 is inserted into the gap 52 between the blocking member 40 and the luer tip 38, and the blocking member 40 is fitted and joined to the inner tubular portion 78.

Thereafter, the user performs operation of alternately pushing the first plunger 24 (see Fig. 2A) of the first syringe 12 and the second plunger 18 (see Fig. 3A) of the second syringe 14. As a result, the first drug A in the first storage chamber 32 and the second drug B in the second storage chamber 72 are mixed, and preparation of an injection C is completed.

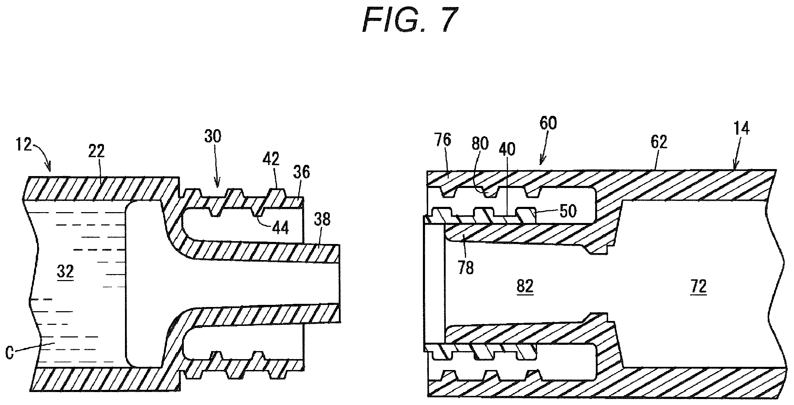

Next, as illustrated in Fig. 7, after the prepared injection C is stored on the first syringe 12 side, the user rotates the second syringe 14 to detach the second syringe 14 from the first syringe 12. In this event, the blocking member 40 rotates integrally with the inner tubular portion 78 of the second connection port 60. The pitch of the male screw 50 of the blocking member 40 is the same as the pitch of the female screw 80 of the outer tubular portion 76 of the second connection port 60, and thus the blocking member 40 is also detached from the needle connection screw 44 of the inner peripheral portion 36b of the lock portion 36 along with release of screwing of the second connection port 60 to the first connection port 30. As a result, the injection needle 16 can be connected to the first connection port 30.

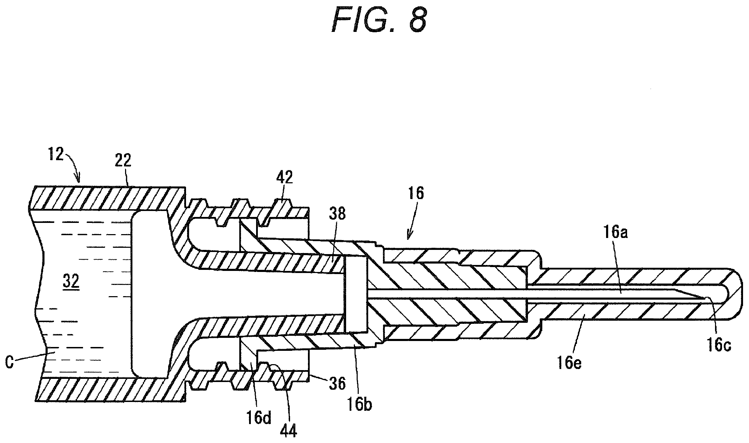

Next, as illustrated in Fig. 8, the injection needle 16 is screwed into the first connection port 30 of the first syringe 12 to connect the injection needle 16, whereby preparation for administration of the injection C is completed. Thereafter, the user removes the needle cap 16e of the injection needle 16, punctures the needle tip 16c subcutaneously, and presses the first plunger 24 (see Fig. 2A) to administer the injection C. Thus, administration of the injection C using the syringe set 10 is completed.

The syringe set 10 of the present embodiment has the following effects.

The syringe set 10 of the present embodiment includes the first syringe 12 and the second syringe 14 (drug container). The first syringe 12 includes the first barrel 22 that stores a liquid first drug A, and the first connection port 30 that is provided at the distal end of the first barrel 22 and communicates with inside of the first barrel 22. In addition, the second syringe 14 stores the second drug B and has the second connection port 60 detachable from the first connection port 30. The first connection port 30 includes the injection needle attachment structure to which the injection needle 16 is attached, and the blocking member 40 provided in the injection needle attachment structure to block attachment of the injection needle 16 to the injection needle attachment structure. When the second connection port 60 is connected to the first connection port 30, the blocking member 40 is connected to the second connection port 60 side, and when the second connection port 60 to which the blocking member 40 is connected is detached from the first connection port 30, the blocking member 40 is detached from the first connection port 30 so as to allow the injection needle 16 to be attached to the injection needle attachment structure.

According to the above configuration, the injection needle 16 cannot be attached to the first syringe 12 unless the second connection port 60 is connected to the first connection port 30 and the first drug A of the first syringe 14 and the second drug B of the second syringe 12 are mixed. This can prevent the user from erroneously administering the unprepared first drug A as a result of attaching the injection needle 16 to the first syringe 12 that stores only the first drug A before the drugs are mixed.

In the syringe set 10, the injection needle attachment structure may include the tubular lock portion 36 in which the needle connection screw 44 into which the injection needle 16 is screwed is formed in the inner peripheral portion 36b, and the luer tip 38 formed inside the lock portion 36 and having the through hole 46 communicating with the inside of the first barrel 22, and the blocking member 40 may be disposed between the lock portion 36 and the luer tip 38. According to this configuration, it is possible to prevent the injection needle 16 from being screwed into the lock portion 36 by the blocking member 40.

In the syringe set 10, the blocking member 40 is formed in a cylindrical shape, and the outer peripheral portion 40c is provided with the male screw 50 as a protrusion to be screwed into the needle connection screw 44 on the inner peripheral side of the lock portion 36. As described above, the blocking member 40 is screwed into the needle connection screw 44, so that it is possible to prevent falling off to the distal end side.

In the syringe set 10 described above, the male screw 42 may be formed on the outer peripheral portion 36a of the lock portion 36 of the first connection port 30, and the second connection port 60 may have the outer tubular portion 76 having, on the inner peripheral portion 76a, the female screw 80 that can be screwed into the male screw 42 of the outer peripheral portion 36a of the lock portion 36, and the inner tubular portion 78 that is formed inside the outer tubular portion 76, has the communication hole 82 communicating with the second storage chamber 72, is inserted between the lock portion 36 and the luer tip 38, and stores the luer tip 38 in the communication hole 82. As a result, the attachment structure of the inner tubular portion 78 and the luer tip 38 is simplified.

In the syringe set 10, the blocking member 40 may be configured such that the inner peripheral portion 40d is separated from the luer tip 38, and the inner tubular portion 78 is inserted into the inner peripheral portion 40d of the blocking member 40 and joined to the blocking member 40. According to this configuration, when the second connection port 60 is connected to the first connection port 30, the blocking member 40 is fitted and joined to the inner tubular portion 78.

In the syringe set 10 described above, the blocking member 40 may block the vicinity of the distal end of the needle connection screw 44 of the inner peripheral portion 36b of the lock portion 36. According to this configuration, the injection needle 16 is reliably prevented from being screwed into the lock portion 36.

In the syringe set 10 described above, the first cap 26 that seals the first syringe 12 may be provided, and the first cap 26 may include the first cap body portion 54b having the twisting portion 54a to be screwed into the male screw 42 of the outer peripheral portion 36a of the lock portion 36, and the sealing member 56 that is supported by the first cap body portion 54b and abuts on the luer tip 38 to seal the through hole 46.

In the syringe set 10, the drug container may be the second syringe 14 having the second barrel 62 as the second storage chamber 72 that stores the second drug B.

In addition, the first syringe 12 of the present embodiment is connected to the drug container that stores the second drug B immediately before use, and administration is performed after the liquid first drug A is mixed with the second drug B. The first syringe 12 includes the first barrel 22 that stores the first drug A, and the first connection port 30 provided at the distal end of the first barrel 22 and configured to be connectable to the drug container (second syringe 14). The first connection port 30 is provided with the injection needle attachment structure to which the injection needle 16 is attachable, and the blocking member 40 that is disposed in the injection needle attachment structure and blocks attachment of the injection needle 16 to the injection needle attachment structure. Then, when the second syringe 14 is connected to the first connection port 30, the blocking member 40 is connected to the second syringe 14 side, and when the second syringe 14 to which the blocking member 40 is connected is detached from the first connection port 30, the blocking member 40 is detached from the first connection port 30 so as to allow the injection needle 16 to be attached to the injection needle attachment structure.

According to the first syringe 12 having the above configuration, attachment of the injection needle 16 in a state of only the unmixed first drug A can be blocked by the blocking member 40, so that erroneous administration can be prevented.

### (Modification of First Embodiment)

In the example described with reference to Fig. 2B, the male screw 50 is provided on the outer peripheral portion 40c of the blocking member 40, and the male screw 50 of the blocking member 40 is screwed into the needle connection screw 44 of the lock portion 36. However, the present embodiment is not limited to this.

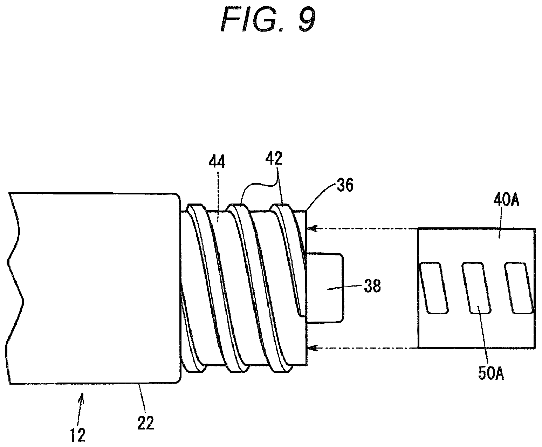

In other words, as illustrated in a separated state in Fig. 9, an outer peripheral portion 40c of a blocking member 40A may be provided with a protrusion 50A that can be screwed into the needle connection screw 44 of the lock portion 36 instead of the male screw 50. The protrusions 50A are provided as protrusions scattered so as to occupy only part of the outer peripheral portion 40c of the blocking member 40 in a circumferential direction. As illustrated in the drawing, a plurality of protrusions 50A may be provided at the same pitch as the pitch of the needle connection screw 44 in the axial direction, or only one protrusion may be provided in the axial direction.

Also by the blocking member 40A of the present modification, the same effects as those of the blocking member 40 can be obtained.

### (Second Embodiment)

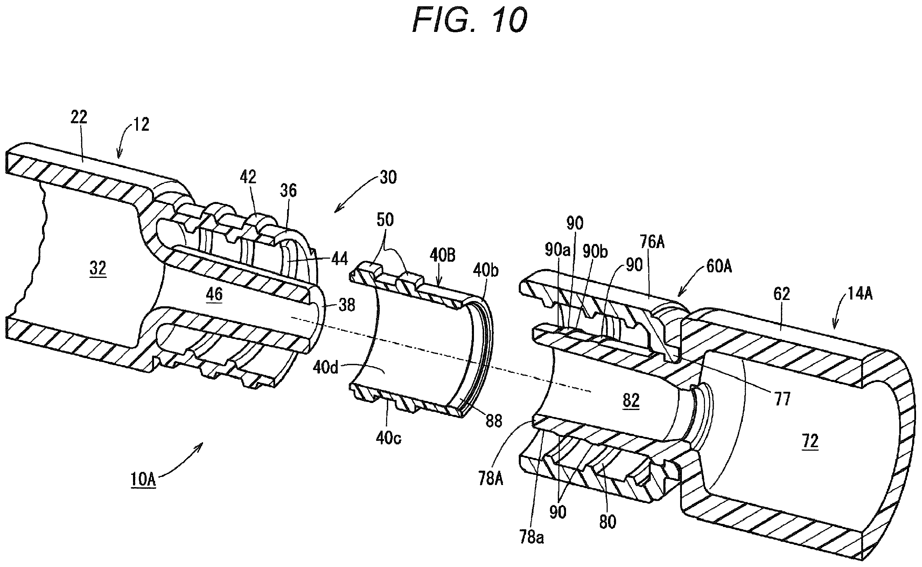

As illustrated in Fig. 10, a syringe set 10A of the present embodiment is different from the syringe set 10 described with reference to Figs. 1 to 9 in that protrusions that can be engaged with each other are provided on an inner peripheral portion 40d of a blocking member 40B and an outer peripheral portion 78a of an inner tubular portion 78A. In the configuration of the syringe set 10A of the present embodiment, the same components as those of the syringe set 10 of the first embodiment are denoted by the same reference numerals, and the detailed description thereof will be omitted.

As illustrated in Fig. 10, the blocking member 40B includes a first protrusion 88 protruding inward from the inner peripheral portion 40d. The first protrusion 88 is provided in the vicinity of a distal end portion 40b of the blocking member 40B and is formed in an annular shape along the circumferential direction of the inner peripheral portion 40d.

In a second connection port 60A of a second syringe 14A, second protrusions 90 are provided on the outer peripheral portion 78a of the inner tubular portion 78A. The second protrusions 90 are annular protrusions protruding outward from the outer peripheral portion 78a of the inner tubular portion 78A and are formed in an annular shape along the circumferential direction of the inner tubular portion 78A. A plurality of the second protrusions 90 is provided at intervals in the axial direction. The second protrusions 90 are formed at a protrusion height that can be engaged with the first protrusion 88 of the blocking member 40B. An inclined surface 90a for smooth insertion into the first protrusion 88 is formed on the distal end side of the second protrusion 90. In addition, on the proximal end side of the second protrusion 90, a vertical surface 90b that stands vertically to the axial direction is provided so as to be hardly detached from the first protrusion 88. The second protrusions 90 do not have to be formed in an entire region in the circumferential direction, and only one second protrusion may be provided in the axial direction.

Although not particularly limited, in the second connection port 60A of the present embodiment, the outer tubular portion 76A is molded separately from the second syringe 14A. The outer tubular portion 76A is assembled to the second syringe 14A by being fitted to a fitting structure 77 provided at the distal end of the second barrel 62 and in the vicinity of a root of the inner tubular portion 78A. The outer tubular portion 76A may be integrally molded with the second syringe 14A similarly to the outer tubular portion 76 (Fig. 3B) of the first embodiment.

The syringe set 10A of the present embodiment is configured as described above, and action thereof will be described below.

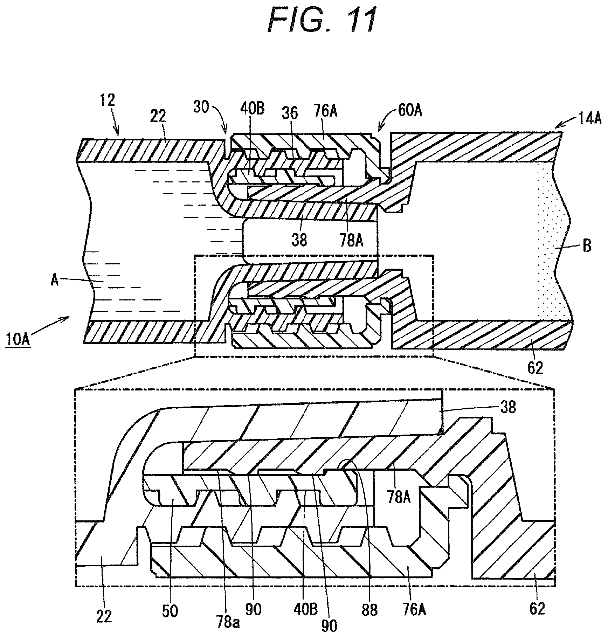

As illustrated in Fig. 11, when the second connection port 60A is connected to the first connection port 30, the inner tubular portion 78A and the second protrusions 90 of the inner tubular portion 78A are inserted into the proximal end side of the first protrusion 88 of the blocking member 40B. When the second connection port 60A is removed from the first connection port 30, the blocking member 40B is pulled out together with the inner tubular portion 78A by the second protrusions 90 being engaged with the first protrusion 88 of the blocking member 40B. Accordingly, the blocking member 40B can be reliably removed together with the second connection port 60A.

The syringe set 10A of the present embodiment has the following effects.

In the syringe set 10A of the present embodiment, protrusions (the first protrusion 88 and the second protrusions 90) that can be engaged with each other are formed on the inner peripheral portion 40d of the blocking member 40B and the outer peripheral portion 78a of the inner tubular portion 78A.

This causes the inner tubular portion 78A to be engaged with the blocking member 40B, so that the blocking member 40B can be more reliably removed.

In the syringe set 10A, the protrusions (the first protrusion 88 and the second protrusions 90) may be formed in an annular shape extending in the circumferential direction. Accordingly, the blocking member 40B can be more reliably removed together with the inner tubular portion 78A.

An uneven structure of the present embodiment is not limited to the annular first protrusion 88 and the second protrusions 90 and may be configured by a combination of an annular groove and a protrusion.

### (Third Embodiment)

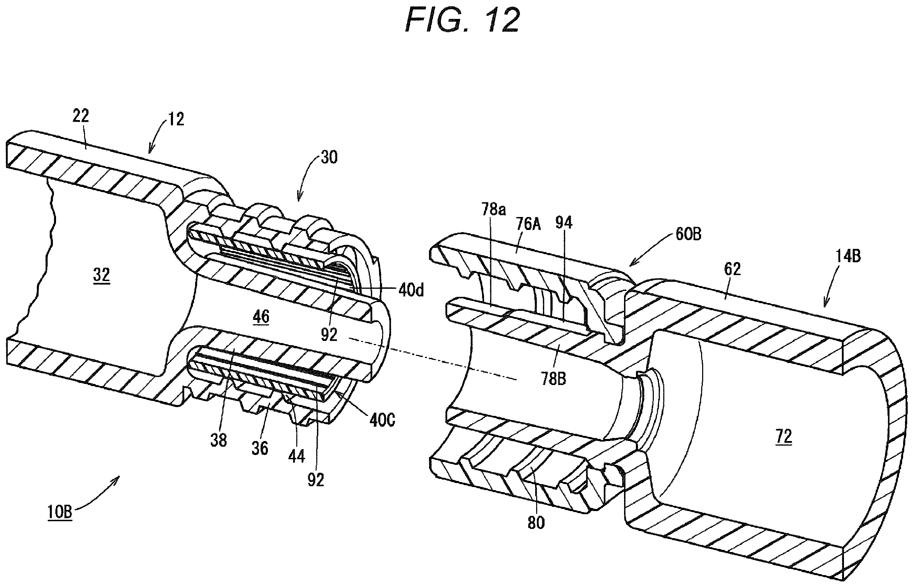

As illustrated in Fig. 12, a syringe set 10B of the present embodiment is provided with uneven structures that can be engaged with each other on an inner peripheral portion 40d of a blocking member 40C and an outer peripheral portion 78a of the inner tubular portion 78B. The uneven structure of the present embodiment is different from the syringe set 10A described with reference to Fig. 10 in including vertical ribs 92 extending in the axial direction formed in the inner peripheral portion 40d of the blocking member 40C. In the configuration of the syringe set 10B of the present embodiment, the same components as those of the syringe set 10A of the second embodiment are denoted by the same reference numerals, and the detailed description thereof will be omitted.

As illustrated in Fig. 12, the vertical ribs 92 extending in the axial direction are formed on the inner peripheral portion 40d of the blocking member 40C. A plurality of vertical ribs 92 is disposed at a constant pitch in the circumferential direction. On the other hand, an enlarged diameter portion 94 protruding radially outward is provided on a proximal end side of the inner tubular portion 78B in the outer peripheral portion 78a of the inner tubular portion 78B of the second syringe 14B.

The syringe set 10B of the present embodiment is configured as described above, and action thereof will be described below.

In the syringe set 10B, when the second connection port 60B is connected to the first connection port 30, the inner tubular portion 78B is inserted into the inner peripheral portion 40d of the blocking member 40C. When the enlarged diameter portion 94 of the inner tubular portion 78B is pushed into the inner peripheral portion 40d of the blocking member 40C, the inner tubular portion 78B and the blocking member 40C are joined by fitting.

Thereafter, when the second connection port 60B is rotated and removed from the first connection port 30, the blocking member 40C is detached together with the inner tubular portion 78B. In this event, the vertical ribs 92 of the blocking member 40C are caught by the inner tubular portion 78B to exert stronger joining force in a rotation direction, and thus, the blocking member 40C rotates integrally with the inner tubular portion 78B, so that the blocking member 40C can be removed more reliably.

As described above, the syringe set 10B of the present embodiment includes the vertical ribs 92 extending in the axial direction as the uneven structure, so that the blocking member 40C can be reliably removed using the needle connection screw 44 of the lock portion 36.

The vertical ribs 92 may be formed on the inner tubular portion 78B side. In addition, the vertical ribs 92 may be formed on both the inner peripheral portion 40d and the inner tubular portion 78B of the blocking member 40C.

### (Fourth Embodiment)

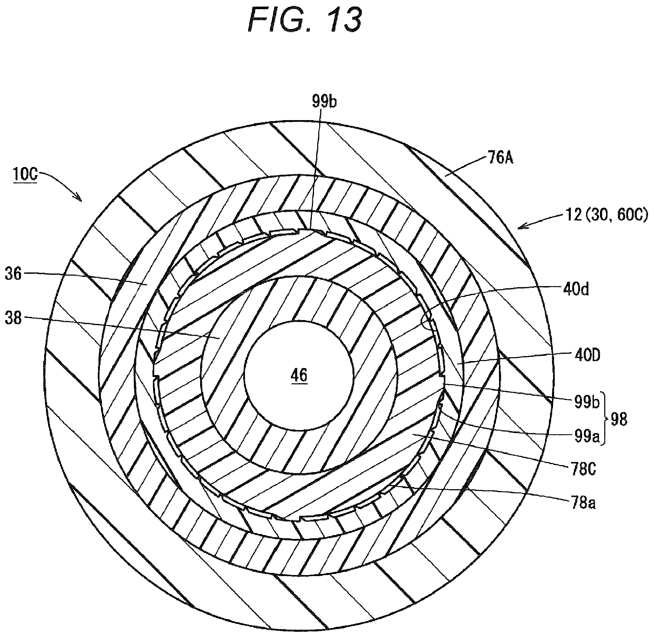

As illustrated in Fig. 13, a syringe set 10C of the present embodiment is provided with uneven structures that can be engaged with each other on the inner peripheral portion 40d of a blocking member 40D and the outer peripheral portion 78a of an inner tubular portion 78C. Fig. 13 illustrates a cross section cut perpendicularly to the axial direction in a state where the second connection port 60C is connected to the first connection port 30. The uneven structure of the present embodiment is different from the syringe set 10A described with reference to Fig. 10 in including a ratchet structure 98 that rotates the blocking member 40D only in a predetermined direction. In the configuration of the syringe set 10C of the present embodiment, the same components as those of the syringe set 10A of the second embodiment are denoted by the same reference numerals, and the detailed description thereof will be omitted.

As illustrated in Fig. 13, in the blocking member 40D of the present embodiment, first ratchet protrusions 99a extending in the axial direction are formed in an inner peripheral portion 40d. A plurality of first ratchet protrusions 99a are provided at regular intervals in the circumferential direction. An inclined surface is formed on one side of a side portion of the first ratchet protrusion 99a.

On the other hand, second ratchet protrusions 99b are formed at a plurality of positions in the circumferential direction on the outer peripheral portion 78a of the inner tubular portion 78C of the second connection port 60C. One side portion of the second ratchet protrusion 99b is formed with an inclined surface.

When the second connection port 60C is screwed into the first connection port 30, the first ratchet protrusions 99a and the second ratchet protrusions 99b come into contact with each other on their inclined surfaces and escape while being elastically deformed in a thickness direction. This enables the user to perform operation of screwing the second connection port 60C.

On the other hand, when the second connection port 60C is rotated in a removal direction, the first ratchet protrusions 99a and the second ratchet protrusions 99b are engaged with each other. As a result, the blocking member 40D rotates integrally with the inner tubular portion 78C and is displaced in a direction away from the first connection port 30 by the needle connection screw 44. As a result, the blocking member 40D is detached from the first connection port 30 together with the inner tubular portion 78C.

As described above, the syringe set 10C of the present embodiment includes, as the uneven structure, the ratchet structure 98 that rotates the blocking member 40D only in a direction of separating from the needle connection screw 44 of the lock portion 36. Accordingly, the blocking member 40D can be reliably removed from the first connection port 30.

### (Fifth Embodiment)

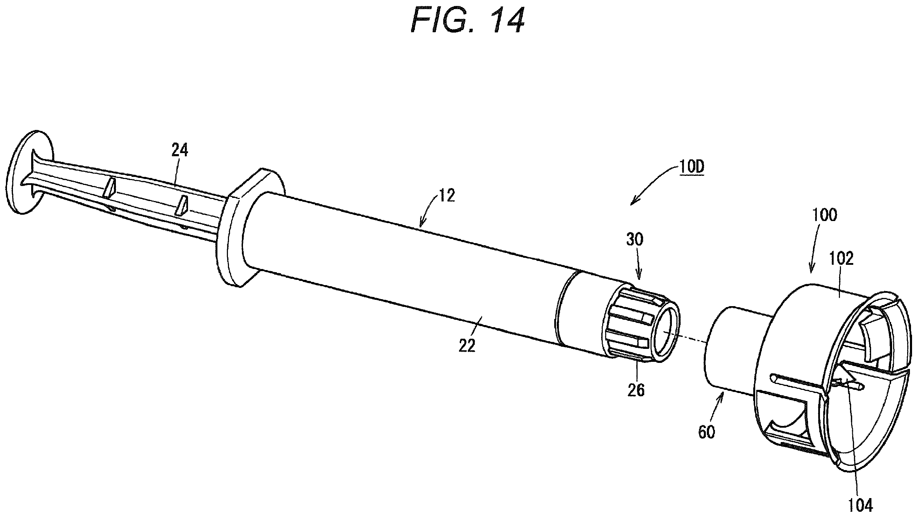

As illustrated in Fig. 14, a syringe set 10D (set) of the present embodiment includes the first syringe 12 and a vial adapter 100 (connector). The syringe set 10D is applied in a case where a vial (not illustrated) is used as a drug container instead of the second syringe 14. In the configuration of the syringe set 10D of the present embodiment, the same components as those of the syringe set 10 described with reference to Figs. 1 to 9 are denoted by the same reference numerals, and a detailed description thereof will be omitted.

As illustrated in the drawing, the vial adapter 100 is a member connected to a bottle opening of the vial container (not illustrated) and includes a connection portion 102 for connecting to the bottle opening of the vial container, a connection needle 104 penetrating a rubber stopper of the vial container, and a second connection port 60 for connecting to the first syringe 12.

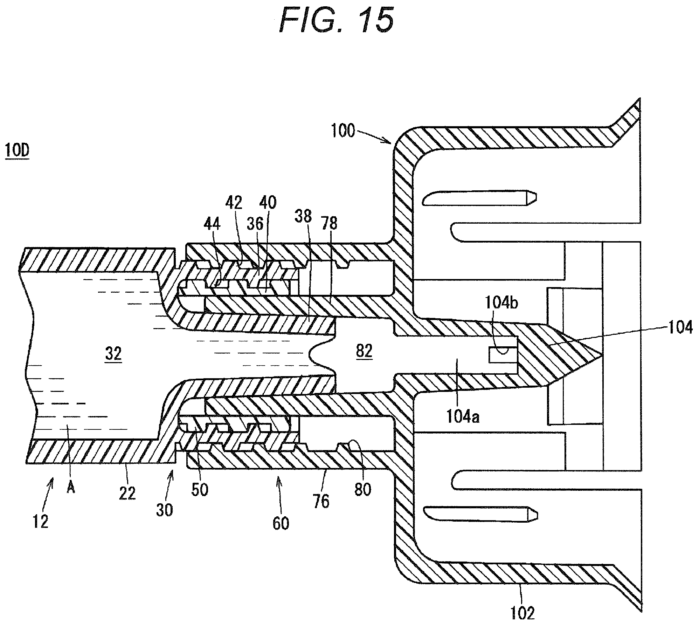

As illustrated in Fig. 15, the connection needle 104 is provided in the vicinity of a central axis of the connection portion 102 and protrudes to one end side of the connection portion 102. A cavity portion 104a is provided at the center of the connection needle 104. The cavity portion 104a communicates with an outer peripheral side of the connection needle 104 through a window portion 104b on a side portion. When the vial container is punctured with the connection needle 104, the cavity portion 104a communicates with inside of the vial container through the window portion 104b.

The second connection port 60 extends from an opposite side of the connection needle 104 across the connection portion 102. A communication hole 82 of the second connection port 60 communicates with the cavity portion 104a of the connection needle 104.

The syringe set 10D of the present embodiment is used by connecting the vial adapter 100 to a vial container and further connecting the first syringe 12 to the vial adapter 100. When the second connection port 60 of the vial adapter 100 is connected to the first connection port 30 of the first syringe 12, the blocking member 40 of the first connection port 30 is joined to the inner tubular portion 78 of the second connection port 60.

Then, when the vial adapter 100 is removed from the first syringe 12 after preparation of the injection, the blocking member 40 is detached from the first connection port 30 together with the vial adapter 100. As a result, the injection needle 16 can be connected to the first syringe 12.

As described above, the syringe set 10D of the present embodiment also prevents erroneous administration due to forgetting of mixing of an injection.

Although the present invention has been described above with reference to the preferred embodiments, the present invention is not limited to the above-described embodiments, and it goes without saying that various modifications can be made without departing from the scope of the invention as defined by the appended claims.

## Claims

1. A syringe set (10, 10A-D) comprising:
a first syringe (12) including a barrel that stores a liquid first drug (A), and a first connection port (30) that is provided at a distal end of the barrel and communicates with inside of the barrel; and
a drug container (14) that stores a second drug (B) and includes a second connection port (60, 60A-C) that is configured to be attachable to and detachable from the first connection port (30),
the first connection port (30) including an injection needle attachment structure to which an injection needle (16) is attachable, and a blocking member (40, 40A-D) that is disposed in the injection needle attachment structure and configured to block attachment of the injection needle (16) to the injection needle attachment structure,
when the second connection port (60, 60A-C) is connected to the first connection port (30), the blocking member (40, 40A-D) being connected to the second connection port side, and
when the second connection port (60, 60A-C) to which the blocking member (40, 40A-D) is connected is detached from the first connection port (30), the blocking member (40, 40A-D) being detached from the first connection port (30) so as to allow the injection needle (16) to be attached to the injection needle attachment structure wherein the injection needle attachment structure comprises: a tubular lock portion (36) in which a needle connection screw (44) into which the injection needle (16) is to be screwed is formed on an inner peripheral portion (36b, 40d, 76a, 78b); and a luer tip (38) formed inside the lock portion (36) and having a through hole (46) communicating with the inside of the barrel, and
the blocking member (40, 40A-D) is disposed between the lock portion (36) and the luer tip (38),
wherein
a male screw (42, 50) is formed on an outer peripheral portion (36a) of the lock portion (36) of the first connection port (30), and
the second connection port (60, 60A-C) comprises:
an outer tubular portion (76, 76A) having a female screw (80) on an inner periphery, the female screw (80) being capable of being screwed into the male screw (42, 50) on the outer periphery portion of the lock portion (36); and
an inner tubular portion (78, 78A-C) that is formed inside the outer tubular portion (76, 76A), has a communication hole (82) communicating with inside of the drug container (14), is inserted between the lock portion (36) and the luer tip (38), and stores the luer tip (38) in the communication hole (82).

2. The syringe set (10, 10A-D) according to claim 1, wherein the blocking member (40, 40A-D) is formed in a cylindrical shape, and a protrusion (50A, 54d, 84c) to be screwed into the needle connection screw (44) of the inner peripheral portion (36b, 40d, 76a, 78b) of the lock portion (36) is provided on an outer peripheral portion (36a).

3. The syringe set (10, 10A-D) according to claim 1, wherein an inner peripheral side of the blocking member (40, 40A-D) is separated from the luer tip (38), and the inner tubular portion (78, 78A-C) is inserted into the inner peripheral side of the blocking member (40, 40A-D) and joined to the blocking member (40, 40A-D).

4. The syringe set (10, 10A-D) according to claim 3, wherein uneven structures engageable with each other are formed on an inner peripheral portion (36b, 40d, 76a, 78b) of the blocking member (40, 40A-D) and an outer peripheral surface of the inner tubular portion (78, 78A-C).

5. The syringe set (10, 10A-D) according to claim 4, wherein the uneven structures include an annular protrusion extending in a circumferential direction.

6. The syringe set (10, 10A-D) according to claim 4, wherein the uneven structures include a vertical rib (92) extending in an axial direction.

7. The syringe set (10, 10A-D) according to claim 4, wherein the uneven structures include a ratchet structure (98) that rotates the blocking member (40, 40A-D) only in a direction in which the blocking member (40, 40A-D) is detached from the needle connection screw (44) of the lock portion (36).

8. The syringe set (10, 10A-D) according to any one of claims 1 to 7, wherein the blocking member (40, 40A-D) blocks a vicinity of a distal end of the needle connection screw (44) in the inner peripheral portion (36b, 40d, 76a, 78b) of the lock portion (36).

9. The syringe set (10, 10A-D) according to claim 1, further comprising a cap that seals the first syringe (12), wherein the cap comprises: a cap body portion having a twisting portion (54a, 84a) to be screwed into the male screw (42, 50) on an outer peripheral portion (36a) of the lock portion (36); and a sealing member (56) that is supported by the cap body portion and abuts on the luer tip (38) to seal the through hole (46).

10. The syringe set (10, 10A-D) according to any one of claims 1 to 9, wherein the drug container (14) is a second syringe (14, 14A-B) having a barrel as a second storage chamber (72) that stores the second drug (B).

## Patentansprüche

1. Spritzenset (10, 10A-D), umfassend:
eine erste Spritze (12), die einen Zylinder, der ein flüssiges erstes Arzneimittel (A) speichert, und einen ersten Verbindungsanschluss (30) aufweist, der an einem distalen Ende des Zylinders vorgesehen ist und mit der Innenseite des Zylinders in Verbindung steht; und
einen Arzneimittelbehälter (14), der ein zweites Arzneimittel (B) speichert und einen zweiten Verbindungsanschluss (60, 60A-C) aufweist, der so konfiguriert ist, dass er an dem ersten Verbindungsanschluss (30) angebracht und von diesem abgenommen werden kann,
wobei der erste Verbindungsanschluss (30) eine Befestigungsstruktur für Injektionsnadeln, an der eine Injektionsnadel (16) anbringbar ist, und ein Blockierungselement (40, 40A-D) aufweist, das in der Befestigungsstruktur für Injektionsnadeln angeordnet und so konfiguriert ist, dass es die Befestigung der Injektionsnadel (16) an der Befestigungsstruktur für Injektionsnadeln blockiert,
wobei, wenn der zweite Verbindungsanschluss (60, 60A-C) mit dem ersten Verbindungsanschluss (30) verbunden ist, das Blockierungselement (40, 40A-D) mit der Seite des zweiten Verbindungsanschlusses verbunden ist, und
wenn der zweite Verbindungsanschluss (60, 60A-C), mit welchem das Blockierungselement (40, 40A-D) verbunden ist, von dem ersten Verbindungsanschluss (30) abgetrennt ist, wird das Blockierungselement (40, 40A-D) von dem ersten Verbindungsanschluss (30) abgetrennt, sodass es der Injektionsnadel (16) gestattet wird, an der Befestigungsstruktur für Injektionsnadeln angebracht zu werden, wobei die Befestigungsstruktur für Injektionsnadeln umfasst: einen röhrenförmigen Verriegelungsabschnitt (36), in dem eine Nadel-Verbindungsschraube (44), in welche die Injektionsnadel (16) einzuschrauben ist, an einem inneren Umfangsabschnitt (36b, 40d, 76a, 78b) ausgebildet ist; und eine Luer-Spitze (38), die im Inneren des Verriegelungsabschnitts (36) ausgebildet ist und ein Durchgangsloch (46) aufweist, das mit der Innenseite des Zylinders in Verbindung steht, und
das Blockierungselement (40, 40A-D) zwischen dem Verriegelungsabschnitt (36) und der Luer-Spitze (38) angeordnet ist, wobei
eine Schraube (42, 50) mit Außengewinde an einem äußeren Umfangsabschnitt (36a) des Verriegelungsabschnitts (36) von dem ersten Verbindungsanschluss (30) ausgebildet ist, und
der zweite Verbindungsanschluss (60, 60A-C) umfasst:
einen äußeren röhrenförmigen Abschnitt (76, 76A), der eine Schraube (80) mit Innengewinde an einem inneren Umfang aufweist, wobei die Schraube (80) mit Innengewinde in der Lage ist, in die Schraube (42, 50) mit Außengewinde an dem äußeren Umfangsabschnitt des Verriegelungsabschnitts (36) geschraubt zu werden; und
ein innerer röhrenförmiger Abschnitt (78, 78A-C), welcher auf der Innenseite des äußeren röhrenförmigen Abschnitts (76, 76A) ausgebildet ist, der ein Verbindungsloch (82) aufweist, das mit der Innenseite des Arzneimittelbehälters (14) in Verbindung steht, zwischen dem Verriegelungsabschnitt (36) und der Luer-Spitze (38) eingesetzt ist und die Luer-Spitze (38) in dem Verbindungsloch (82) aufbewahrt.

2. Spritzenset (10, 10A-D) nach Anspruch 1, wobei das Blockierungselement (40, 40A-D) in einer zylindrischen Form ausgebildet ist und ein Vorsprung (50A, 54d, 84c), der in die Nadel-Verbindungsschraube (44) des inneren Umfangsabschnitts (36b, 40d, 76a, 78b) des Verriegelungsabschnitts (36) zu schrauben ist, an einem äußeren Umfangsabschnitt (36a) vorgesehen ist.

3. Spritzenset (10, 10A-D) nach Anspruch 1, wobei eine innere Umfangsseite des Blockierungselements (40, 40A-D) von der Luer-Spitze (38) getrennt ist, und der innere röhrenförmige Abschnitt (78, 78A-C) in die innere Umfangsseite des Blockierungselements (40,40A-D) eingesetzt und mit dem Blockierungselement (40,40A-D) verbunden ist.

4. Spritzenset (10, 10A-D) nach Anspruch 3, wobei ungleichmäßige Strukturen, die miteinander in Eingriff bringbar sind, an einem inneren Umfangsabschnitt (36b, 40d, 76a, 78b) des Blockierungselements (40, 40A-D) und einer äußeren Umfangsfläche des inneren röhrenförmigen Abschnitts (78, 78A-C) ausgebildet sind.

5. Spritzenset (10, 10A-D) nach Anspruch 4, wobei die ungleichmäßigen Strukturen einen ringförmigen Vorsprung umfassen, der sich in einer Umfangsrichtung erstreckt.

6. Spritzenset (10, 10A-D) nach Anspruch 4, wobei die ungleichmäßigen Strukturen eine senkrechte Rippe (92) umfassen, die sich in einer axialen Richtung erstreckt.

7. Spritzenset (10, 10A-D) nach Anspruch 4, wobei die ungleichmäßigen Strukturen eine Ratschen-Struktur (98) umfassen, welche das Blockierungselement (40, 40A-D) nur in eine Richtung dreht, in der das Blockierungselement (40, 40A-D) von der Nadel-Verbindungsschraube (44) des Verriegelungsabschnitts (36) abgetrennt wird.

8. Spritzenset (10, 10A-D) nach einem der Ansprüche 1 bis 7, wobei das Blockierungselement (40, 40A-D) eine Umgebung eines distalen Endes der Nadel-Verbindungsschraube (44) in dem inneren Umfangsabschnitts (36b, 40d, 76a, 78b) des Verriegelungsabschnitts (36) blockiert.

9. Spritzenset (10, 10A-D) nach Anspruch 1, ferner umfassend eine Kappe, welche die erste Spritze (12) abdichtet, wobei die Kappe umfasst: einen Kappenkörperabschnitt, der einen Drehabschnitt (54a, 84a) aufweist, welcher in die Schraube (42, 50) mit Außengewinde an einem äußeren Umgebungsabschnitt (36a) des Verriegelungsabschnitts (36) zu schrauben ist; und ein Dichtungselement (56), welches durch den Kappenkörperabschnitt gestützt wird und an der Luer-Spitze (38) anliegt, um das Durchgangsloch (46) abzudichten.

10. Spritzenset (10, 10A-D) nach einem der Ansprüche 1 bis 9, wobei der Arzneimittelbehälter (14) eine zweite Spritze (14, 14A-B) ist, die einen Zylinder als eine zweite Speicherkammer (72) aufweist, welche das zweite Arzneimittel (B) speichert.

## Revendications

1. Ensemble seringue (10, 10A-D) comprenant :
une première seringue (12) comprenant un cylindre qui stocke un premier médicament liquide (A), et un premier orifice de connexion (30) situé à une extrémité distale du cylindre et communiquant avec l'intérieur du cylindre ; et
un contenant de médicament (14) qui stocke un second médicament (B) et qui comprend un second orifice de connexion (60, 60A-C) configuré pour pouvoir être fixé au premier orifice de connexion (30) et détaché de celui-ci,
le premier orifice de connexion (30) comprenant une structure de fixation d'aiguille d'injection à laquelle une aiguille d'injection (16) peut être fixée, et un élément de blocage (40, 40A-D) disposé dans la structure de fixation d'aiguille d'injection et configuré pour bloquer la fixation de l'aiguille d'injection (16) à la structure de fixation d'aiguille d'injection,
lorsque le second port de connexion (60, 60A-C) est connecté au premier port de connexion (30), l'élément de blocage (40, 40A-D) étant connecté au second côté port de connexion, et
lorsque le second orifice de connexion (60, 60A-C) auquel l'élément de blocage (40, 40A-D) est connecté est détaché du premier orifice de connexion (30), l'élément de blocage (40, 40A-D) étant détaché du premier orifice de connexion (30) de manière à permettre à l'aiguille d'injection (16) d'être fixée à la structure de fixation de l'aiguille d'injection, la structure de fixation d'aiguille d'injection comprenant : une section de verrouillage tubulaire (36) dans laquelle une vis de connexion d'aiguille (44) dans laquelle l'aiguille d'injection (16) doit être vissée est formée sur une section périphérique intérieure (36b, 40d, 76a, 78b) ; et une pointe luer (38) est formée à l'intérieur de la section de verrouillage (36) et ayant un trou traversant (46) communiquant avec le côté intérieur du cylindre, et
l'élément de blocage (40, 40A-D) étant disposé entre la section de verrouillage (36) et la pointe luer (38),
une vis mâle (42, 50) étant formée sur une section périphérique extérieure (36a) de la section de verrouillage (36) du premier orifice de connexion (30), et
le second port de connexion (60, 60A-C) comprenant :
une section tubulaire externe (76, 76A) ayant une vis femelle (80) sur une périphérie interne, la vis femelle (80) pouvant être vissée dans la vis mâle (42, 50) sur la section périphérique externe de la section de verrouillage (36) ; et
une section tubulaire interne (78, 78A-C) formée à l'intérieur de la section tubulaire externe (76, 76A), présentant un trou de communication (82) communiquant avec l'intérieur du récipient de médicament (14), étant insérée entre la section de verrouillage (36) et la pointe luer (38), et stockant la pointe luer (38) dans le trou de communication (82).

2. Ensemble seringue (10, 10A-D) selon la revendication 1, dans lequel l'élément de blocage (40, 40A-D) a une forme cylindrique, et une saillie (50A, 54d, 84c) destinée à être vissée dans la vis de connexion d'aiguille (44) de la section périphérique intérieure (36b, 40d, 76a, 78b) de la section de verrouillage (36) est prévue sur une section périphérique extérieure (36a).

3. Ensemble seringue (10, 10A-D) selon la revendication 1, dans lequel un côté périphérique intérieur de l'élément de blocage (40, 40A-D) est séparé de la pointe luer (38), et la section tubulaire intérieure (78, 78A-C) est insérée dans le côté périphérique intérieur de l'élément de blocage (40, 40A-D) et jointe à l'élément de blocage (40, 40A-D).

4. Ensemble seringue (10, 10A-D) selon la revendication 3, dans lequel des structures irrégulières pouvant s'engager les unes dans les autres sont formées sur une section périphérique intérieure (36b, 40d, 76a, 78b) de l'élément de blocage (40, 40A-D) et une surface périphérique extérieure de la section tubulaire intérieure (78, 78A-C).

5. Ensemble seringue (10, 10A-D) selon la revendication 4, dans lequel les structures irrégulières comprennent une saillie annulaire s'étendant dans une direction circonférentielle.

6. Ensemble seringue (10, 10A-D) selon la revendication 4, dans lequel les structures irrégulières comprennent une nervure verticale (92) s'étendant dans une direction axiale.

7. Ensemble seringue (10, 10A-D) selon la revendication 4, dans lequel les structures irrégulières comprennent une structure à cliquet (98) qui fait tourner l'élément de blocage (40, 40A-D) uniquement dans une direction dans laquelle l'élément de blocage (40, 40A-D) est détaché de la vis de connexion d'aiguille (44) de la section de verrouillage (36).

8. Ensemble seringue (10, 10A-D) selon l'une quelconque des revendications 1 à 7, dans lequel l'élément de blocage (40, 40A-D) bloque un voisinage d'une extrémité distale de la vis de connexion d'aiguille (44) dans la section périphérique intérieure (36b, 40d, 76a, 78b) de la section de verrouillage (36).

9. Ensemble seringue (10, 10A-D) selon la revendication 1, comprenant en outre un capuchon qui ferme hermétiquement la première seringue (12), le capuchon comprenant : une section de corps de capuchon ayant une section de torsion (54a, 84a) destinée à être vissée dans la vis mâle (42, 50) sur une section périphérique extérieure (36a) de la section de verrouillage (36) ; et un élément d'étanchéité (56) qui est supporté par la section de corps de capuchon et bute sur la pointe luer (38) pour fermer hermétiquement le trou traversant (46).

10. Ensemble seringue (10, 10A-D) selon l'une quelconque des revendications 1 à 9, dans lequel le récipient de médicament (14) est une seconde seringue (14, 14A-B) ayant un cylindre servant de seconde chambre de stockage (72) qui stocke le second médicament (B).
